(19) Europäisches Patentamt
European Patent Office
Office européen des brevets

(11) Veröffentlichungsnummer: **0 680 956 A1**

# (12) EUROPÄISCHE PATENTANMELDUNG

(21) Anmeldenummer: **95102962.8**

(22) Anmeldetag: **02.03.95**

(51) Int. Cl.[6]: **C07D 291/08**, C07B 39/00

(30) Priorität: **15.03.94 DE 4408681**

(43) Veröffentlichungstag der Anmeldung: **08.11.95 Patentblatt 95/45**

(84) Benannte Vertragsstaaten: **CH DE FR GB IT LI NL**

(71) Anmelder: **HOECHST AKTIENGESELLSCHAFT**
**Brüningstrasse 50**
**D-65929 Frankfurt am Main (DE)**

(72) Erfinder: **Cabrera, Ivan, Dr.**
**Mühlweg 14**
**D-63303 Dreieichenhain (DE)**
Erfinder: **Appel, Wolfgang, Dr.**
**Taunusstrasse 74a**
**D-65779 Kelkheim (DE)**

(54) **Benz-1,2,3-oxathiazin-4(3F)-on-2,2-dioxide, ein Verfahren zu ihrer Herstellung und ihre Verwendung als elekfrophile Fluorierungsmittel.**

(57) Die Erfindung betrifft Verbindungen der allgemeinen Formel (I)

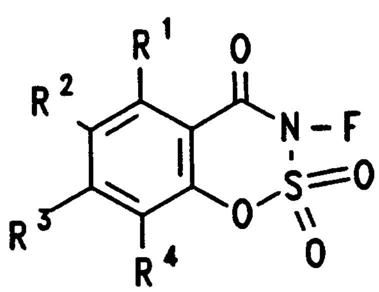

(I)

worin $R^1$, $R^2$, $R^3$ und $R^4$ unabhängig voneinander Wasserstoff, $CF_3$, $NO_2$, CN, Halogen, $-S(NSO_2CF_3)CF_3$, $(C_1-C_4)$-Alkyl, Phenyl, $SO_2R^5$, $COOR^5$, $NR_2{}^5$, wobei $R^5$ $(C_1-C_4)$-Alkyl, das auch fluoriert sein kann oder Phenyl bedeutet, sind, oder $R^1$ und $R^2$, $R^2$ und $R^3$ oder $R^3$ und $R^4$ zusammen einen aliphatischen oder aromatischen Ring bilden, der auch ein Sauerstoff-, Schwefel- oder Stickstoffatom enthalten kann.

Die Erfindung betrifft ferner ein Verfahren zur Herstellung der Verbindungen der Formel (I), indem man Verbindungen der allgemeinen Formel (II)

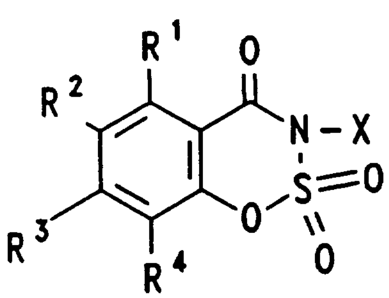

(II)

EP 0 680 956 A1

worin $R^1$ bis $R^4$ die angegebene Bedeutung besitzen und X für Wasserstoff oder ein Alkalimetall steht, in Gegenwart eines inerten Lösungsmittels und gegebenenfalls eines Alkalimetallfluorids bei niedrigen Temperaturen mit elementarem Fluor umsetzt.

Die Erfindung betrifft weiterhin die Verwendung der Verbindungen der Formel (I) zur Fluorierung von Verbindungen mit Carbanioncharakter.

Die Erfindung betrifft Benz-1,2,3-oxathiazin-4(3F)-on-2,2-dioxide, ein Verfahren zu ihrer Herstellung und ihre Verwendung als elektrophile Fluorierungsmittel.

Viele neue Agro- und Pharmawirkstoffe enthalten F-Atome an strategischen Positionen. Ein Grund hierfür ist die Tatsache, daß der Ersatz von Wasserstoff durch Fluor (isosterische Substitution) oder von Hydroxylgruppen durch Fluor (isopolare Substitution) sehr oft zu einer Verbesserung der Wirksamkeit führt. Die selektive Einführung von Fluor in organische Moleküle ist deshalb eine sehr wichtige Aufgabe in der modernen Chemie geworden. Obwohl durch die Einführung von Diethylamino-Schwefeltrifluorid und anderer Reagenzien dieser Verbindungsklasse ein Durchbruch auf dem Gebiet der nukleophilen Fluorierung erreicht wurde, besteht weiterer Bedarf an sicheren, milden und effizienten elektrophilen Fluorierungsmitteln. Die meisten solcher elektrophilen Reagenzien, wie z. B. Perchlorylfluorid, Trifluormethylhypofluorid, $CsSO_4F$ usw. sind toxische und sehr aggressive Chemikalien, mit denen nicht selten Explosionen beobachtet wurden. Darüberhinaus ist die Lagerstabilität solcher Materialien sehr begrenzt. "$F^{\oplus}$"-Reagenzien auf der Basis von N-F haltigen Verbindungen wurden sehr intensiv untersucht, da einige dieser Materialien sich als gut isolierbar, lagerstabile und effiziente Fluorierungsmitteln bewährt haben. Erste Versuche in dieser Richtung wurden mit Perfluor-N-Fluorpiperidin (A) (J. Chem. Soc. Perkin Trans. I 1988, 2805) durchgeführt. Aufgrund der aufwendigen Synthese (Ausbeute max. 13 %) und der Nebenreaktion während der Fluorierung ist diese Verbindung für praktische Zwecke jedoch nicht von Interesse. Andere bekannte N-F Fluorierungsmittel sind N-Fluorpyridin-2-(1H)one (B) (J. Org. Chem. 1983, 43, 761), N-Fluorsulfonamide (C) (U.S. Pat. 4,479,901, 4,828,764, DE 36 23 184 A); Camphor-N-Fluor-Sultam (D) (Tetrahedron Lett. 1988, 29, 6087); N-Fluorquinicludinium Salze (E) (J. Chem. Soc. Perkin Trans I, 1988, 2805); N-Fluorpyridinium Salze (F) (J. Am. Chem. Soc. 1990, 112, 8563); N-Fluor-N-Perfluormethyl Sulfonamides (G) (U.S. Pat. 4,828,764, U.S. Pat. 5,227,493) und N-Fluor-N-chlormethyl-triethylendiamin-bis(tetrafluorborat) (F-Teda (H)) (U.S. Pat. 5,086,178).

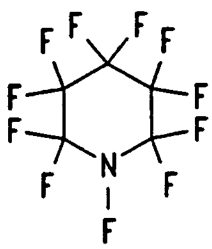
(A)

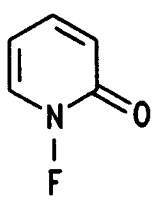
(B)

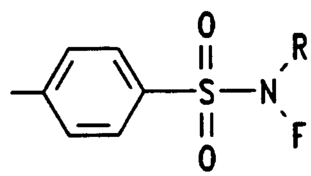 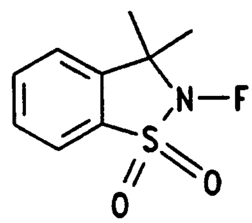
(C)

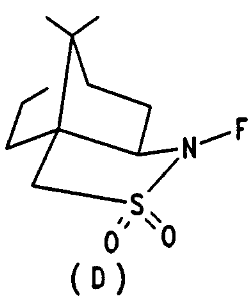
(D)

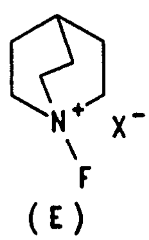
(E)

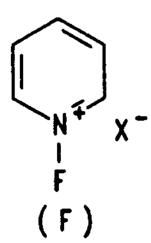
(F)

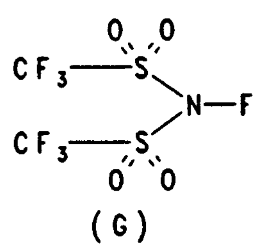
(G)

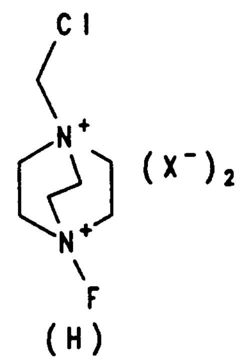
(H)

Die Verbindung (B) ist nicht lagerstabil. Das Reagenz (G), das die stärkste bekannte NF-Verbindung darstellt, erfordert eine sehr aufwendige Synthese für seine Herstellung. Die Verbindungen (C), (F) und (H) sind kommerziell erhältlich. Die käuflichen N-Flurosulfonamide haben allerdings den Nachteil, daß aufgrund des Wasserstoffatoms an der $\alpha$-Stelle des N-Alkylrests eine HF-Eliminierung sehr leicht als Nebenreaktion auftreten kann. Mit N-Alkylresten, die keine Wasserstoffatome in der $\alpha$-Stelle besitzen, z. B. einer t-Butylgruppe, ist die Ausbeute bei der Herstellung der NF-Verbindung sehr gering. Obwohl die geladenen Systeme (H), (F), sehr effiziente Fluorierungsmittel sind, ist ein entscheidender Nachteil dieser Systeme ihre begrenzte Löslichkeit in den gängigen organischen Lösungsmitteln. F-Teda (H) hat zusätzlich den Nachteil, daß bei diesem quaternären Ammoniumsalz oft eine Hofmann-Eliminierung stattfindet. Dies ist besonders

problematisch bei der Fluorierung von starken Carbanionen.

Es bestand daher ein großer Bedarf nach einem elektrophilen Fluorierungsmittel, das die beschriebenen Nachteile nicht aufweist, aus gut zugänglichen Edukten einfach herzustellen ist und hohe Lagerstabilität besitzt.

Diese Aufgabe wird gelöst durch Verbindungen der allgemeinen Formel (I)

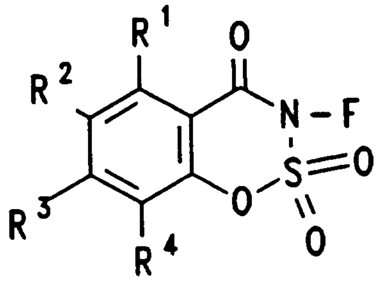

(I)

worin $R^1$, $R^2$, $R^3$ und $R^4$ unabhängig voneinander Wasserstoff, $CF_3$, $NO_2$, CN, Halogen, $-S(NSO_2CF_3)CF_3$, $(C_1-C_4)$-Alkyl, Phenyl, $SO_2R^5$, $COOR^5$, $NR_2{}^5$, wobei $R^5$ $(C_1-C_4)$-Alkyl, das auch fluoriert sein kann oder Phenyl bedeutet, sind, oder $R^1$ und $R^2$, $R^2$ und $R^3$ oder $R^3$ und $R^4$ zusammen einen aliphatischen oder aromatischen Ring bilden, der auch ein Sauerstoff-, Schwefel- oder Stickstoffatom enthalten kann.

Von Interesse sind hierbei in vielen Fällen Verbindungen der Formel (I), worin $R^1$, $R^2$, $R^3$ und $R^4$ unabhängig voneinander Wasserstoff, $NO_2$, CN, $CF_3$, $SO_2R^5$, $S(NSO_2CF_3)CF_3$ bedeuten.

Wichtig sind dabei besonders die di- und monosubstituiterten Verbindungen, d.h. Verbindungen der Formel (I), worin 2 oder 3 der Reste $R^1$, $R^2$, $R^3$, $R^4$ Wasserstoff bedeuten.

Eine gewisse Bedeutung kommt auch den Verbindungen der Formel (I) zu, worin $R^1$ und $R^2$ oder $R^2$ und $R^3$ oder $R^3$ und $R^4$ einen Phenyl oder Cyclohexylring bilden.

Besonders interessant ist natürlich auch die unsubstituierte Verbindung, Benz-1,2,3-oxathiazin-4(3F)-on-2,2-dioxid.

Abhängig vom Substituentenmuster und vom Lösungsmittel ist es auch möglich, daß die Verbindungen der Formel (I) im tautomeren Gleichgewicht mit den Verbindungen der Formel (I') stehen:

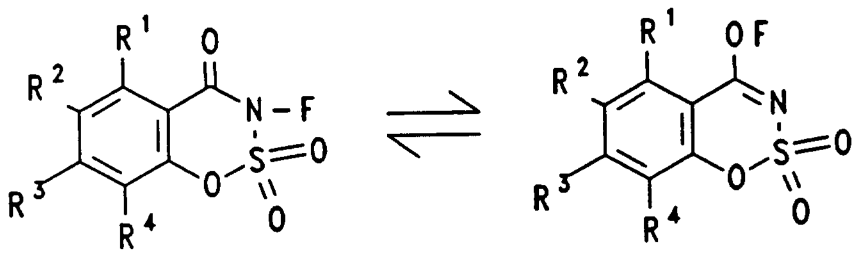

(I) (I')

Die Erfindung betrifft ferner ein Verfahren zur Herstellung der Verbindungen der Formel (I). Es ist dadurch gekennzeichnet, daß man Verbindungen der Formel (II) (hergestellt nach Angew. Chem. 85, 965 (1973)), worin $R^1$, $R^2$, $R^3$ und $R^4$ die oben erwähnte Bedeutung besitzen, und X für Wasserstoff oder ein Alkalimetall steht, in Gegenwart eines inerten Lösungsmittels und gegebenenfalls eines Alkalimetallfluorids bei tiefen Temperaturen mit elementarem Fluor umsetzt.

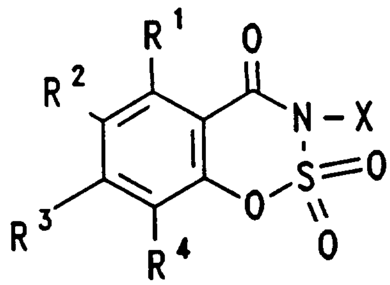

(II)

Gute Ergebnisse werden z. B. erhalten, wenn X für Wasserstoff, Natrium oder Kalium steht.

Als Alkalimetallfluoride haben sich Natrium- oder Kaliumfluorid, insbesondere Natriumfluorid bewährt.

Das Fluorierungsmittel Fluor wird vorteilhaft im Gemisch mit Inertgas wie Stickstoff, $SF_6$, $CF_4$ oder Edelgasen wie Helium, Neon, Argon, Krypton eingesetzt. Bevorzugtes Inertgas ist Stickstoff. Für die Fluorierung können Fluor/Inertgas-Gemische eingesetzt werden, die bis zu 30 Vol.-% Fluor enthalten.

Es hat sich in vielen Fällen bewährt, die Fluorierung mit einem $N_2/F_2$-Gemisch vorzunehmen, das zwischen 1 und 15 Vol.-%, insbesondere 2 und 10 Vol.-%, bevorzugt 3 bis 6 Vol.-% $F_2$ enthält.

Als Lösungsmittel sind z. B. Halogenkohlenwasserstoffe wie Dichlormethan, Chloroform, Tetrachlorkohlenstoff, Fluortrichlormethan, Trifluor-Trichloräthan, Tetrafluor-Dichloräthan oder Nitrile, insbesondere Acetonitril geeignet.

Die Temperatur bei der die Umsetzung durchgeführt wird, kann in weiten Bereichen variiert werden und liegt insbesondere im Bereich von +10 bis -80 °C. Die Wahl der Temperatur hängt im Einzelfall von der Wahl der Reaktionsbedingungen wie Fluorkonzentration, Zusammensetzung des Lösungsmittelgemisches etc. ab.

Die Fluorierung wird vorteilhaft bei Temperaturen von -80 bis -20 °C, insbesondere -60 bis -30 °C, bevorzugt -50 bis -35 °C durchgeführt.

Die Erfindung betrifft weiterhin die Verwendung der Verbindungen der Formel (I) zur Fluorierung von Verbindungen die offenen oder versteckten Carbanion-Charakter besitzen, wie z. B. 1,3-Dicarbonylverbindungen. Diese Verbindungen können in sehr hohen Ausbeuten zu den Fluorverbindungen umgesetzt werden.

Eine Übersicht über derartige Reaktion gibt Tabelle 1

Tabelle 1

| Bsp. | Edukt | Produkt | Ausbeute (%) |
|---|---|---|---|
| 2 | 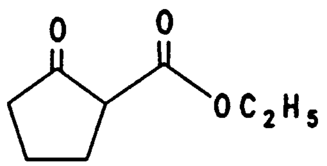 | 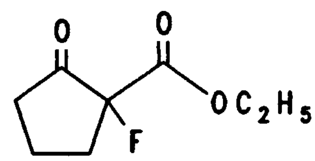 | 77 |
| 3 | 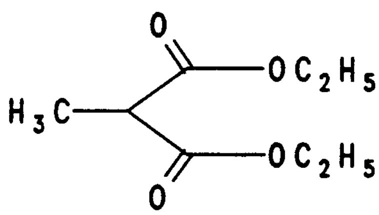 | 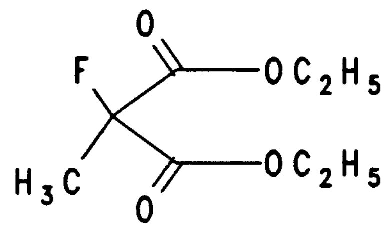 | 78 |
| 4 | 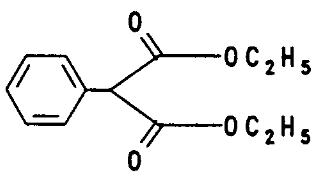 | 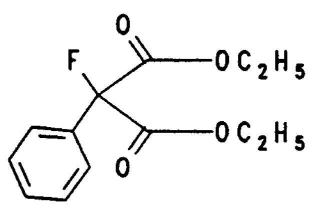 | 86 |
| 5 | 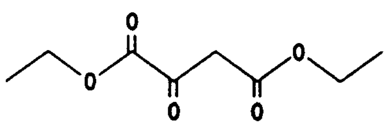 | 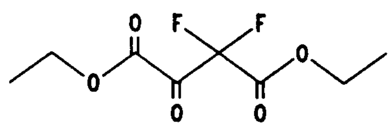 | 65 |
| 6 | 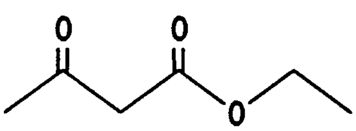 | 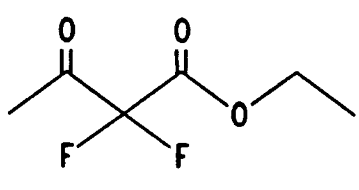 | 70 |

Die Verbindungen der Formel (I) sind in der Lage Iod zu erzeugen, wenn man sie mit Natriumiodidlösung behandelt. Die Konzentration der Verbindungen kann deshalb mittels Titration festgestellt werden.

Ein weiterer wesentlicher Vorteil der neuen Verbindungen beruht auf ihren Löslichkeitseigenschaften: während die NF-Verbindungen gut in organischen Lösungsmitteln löslich sind, sind die Ausgangsmaterialien wasserlöslich. Dies bedeutet, daß die nach einer Fluorierungsreaktion entstandenen N-X-Verbindunen gut mit Wasser aus dem Reaktionsmedium entfernt werden können. Dies resultiert in einer einfachen Isolierung der gewünschten fluorierten Produkte. Darüber hinaus ist deshalb eine einfach Recyclisierung

des hier beschriebenen Materialen möglich, da die N-X-Verbindungen die Ausgangsmaterialien für die Herstellung der NF-Verbindungen sind.

Beispiel 1 Herstellung von Benz-1,2,3-oxathiazin-4(3F)-on-2,2-dioxid

In der vollständig trockenen (ausgeflammten) unter $N_2$ stehenden Fluorierungsapparatur werden 0,98 g (4,4 mmol) 1,2,3-Benz-oxathiazin-4(3H)-on-2,2-dioxid Natriumsalz in 120 ml mit $CaH_2$ getrocknetem Acetonitril suspendiert, mit 0,20 g NaF versetzt und bei -40 °C mit einer 5 % (v/v) $F_2$ in $N_2$-Mischung fluoriert. Anschließend wird 0,5 Stunden bei -40 °C und 1 Stunde bei Raumtemperatur mit $N_2$ gespült. Das Gemisch wird filtriert und das Filtrat an der Ölpumpe schnell abdestilliert. Der weiß-gelbliche Feststoff wird in getrocknetem Ether aufgenommen, klar filtriert und am Rotationsdampfer eingeengt. Das Material kann weiter gereinigt werden durch Ausfällen aus Etherlösung mit Pentan (purum). Kristallines Benz-1,2,3-oxathiazin-4(3F)-on-2,2-dioxid wird abfiltriert und mit Pentan gewaschen. Nach dem Trocknen an der Ölpumpe erhält man 0,85 g Benz-1,2,3-oxathiazin-4(3F)-on-2,2-dioxid (88 % d. Th. bezogen auf 11b). Schmp. 52 bis 53 °C.

$^1$H-NMR (300 MHz, $CDCl_3$): $\delta$ (ppm) 7,42 (dd, 1 H), 7,59 (dt, 1 H), 7,85 (dt, 1 H), 8,23 (dd, 1 H).

Beispiele der Verbindungen, die mit Benz-1,2,3-oxathiazin-4(3F)-on-2,2-dioxid hergestellt wurden, sind in der Tabelle 1 zusammengefaßt. Die Synthese für Beispiel 2 ist nachfolgend beschrieben. Die übrigen Verbindungen werden analog erhalten.

Beispiel 2 Cyclopentanon-2-fluor-2-carbonsäureethylester

Eine Lösung von 187 mg (1,2 mmol) Cyclopentanon-2-carbonsäureethylester in 50 ml getrocknetem THF wird zu einer Suspension von 36 mg NaH (in 10 ml THF) bei 0 °C unter $N_2$ zugegeben. Die Mischung wird 0,5 Stunden bei 0 °C und 1 Stunde bei Raumtemperatur gerührt, bevor das Fluorierungsmittel Benz-1,2,3-oxathiazin-4(3F)-on-2,2-dioxid (302 mg, 1,3 mmol in 10 ml THF) zugegeben wird. Die Reaktionsmischung wird 1 Stunde bei Raumtemperatur gerührt. Das Lösungsmittel wird am Rotationsdampfer abgezogen und der Rückstand in Ether aufgenommen, klar filtriert und das Filtrat mit Wasser, gesät. $NaHCO_3$ Lösung und Wasser ausgeschüttelt. Die Etherphase wird dann mit $Na_2SO_4$ getrocknet, abfiltriert und das Lösungsmittel unter Vakuum entfernt. Ausbeute 161 mg (77 % d. Th.)

**Patentansprüche**

**1.** Verbindungen der allgemeinen Formel (I)

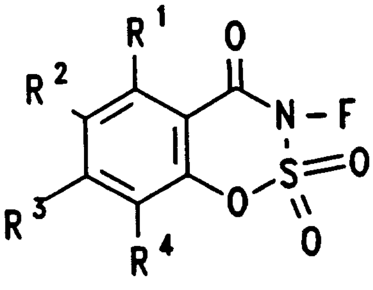

(I)

worin $R^1$, $R^2$, $R^3$ und $R^4$ unabhängig voneinander Wasserstoff, $CF_3$, $NO_2$, CN, Halogen, $-S(NSO_2CF_3)-CF_3$, $(C_1-C_4)$-Alkyl, Phenyl, $SO_2R^5$, $COOR^5$, $NR_2{}^5$, wobei $R^5$ $(C_1-C_4)$-Alkyl, das auch fluoriert sein kann oder Phenyl bedeutet, sind,
oder $R^1$ und $R^2$, $R^2$ und $R^3$ oder $R^3$ und $R^4$ zusammen einen aliphatischen oder aromatischen Ring bilden, der auch ein Sauerstoff-, Schwefel- oder Stickstoffatom enthalten kann.

**2.** Verbindungen der allgemeinen Formel (I), worin $R^1$, $R^2$, $R^3$ und $R^4$ unabhängig voneinander Wasserstoff, $NO_2$, CN, $CF_3$ $SO_2R^5$, $S(NSO_2CF_3)CF_3$ bedeuten.

**3.** Verbindungen nach Anspruch 1 oder 2, worin 2 der Reste $R^1$, $R^2$, $R^3$, $R^4$ Wasserstoff bedeuten.

**4.** Verbindungen nach Anspruch 1 oder 2, worin 3 der Reste $R^1$, $R^2$, $R^3$, $R^4$ Wasserstoff bedeuten.

**5.** Verbindungen der Formel (I) zu, worin $R^1$ und $R^2$ oder $R^2$ und $R^3$ oder $R^3$ und $R^4$ einen Phenyl oder Cyclohexylring bilden.

**6.** Benz-1,2,3-oxathiazin-4(3F)-on-2,2-dioxid.

**7.** Verfahren zur Herstellung der Verbindung nach Anspruch 1 bis 6, dadurch gekennzeichnet, daß man Verbindungen der allgemeinen Formel (II)

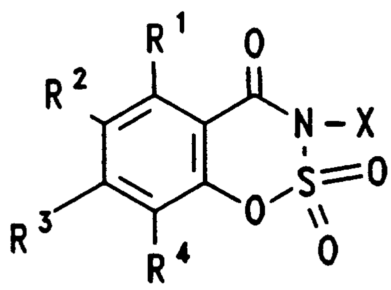

(II)

worin $R^1$ bis $R^4$ die angegebene Bedeutung besitzen und X für Wasserstoff oder ein Alkalimetall steht, in Gegenwart eines inerten Lösungsmittels und gegebenenfalls eines Alkalimetallfluorids bei niedrigen Temperaturen mit elementarem Fluor umsetzt.

**8.** Verfahren nach Anspruch 7, dadurch gekennzeichnet, daß X für Wasserstoff, Natrium oder Kalium steht.

**9.** Verfahren nach Anspruch 7 oder 8, dadurch gekennzeichnet, daß als Alkalimetallfluorid Natrium- oder Kaliumfluorid, insbesondere Natriumfluorid eingesetzt wird.

**10.** Verfahren nach mindestens einem der Ansprüche 7 bis 9, dadurch gekennzeichnet, daß das Fluorierungsmittel Fluor im Gemisch mit Inertgasen wie Stickstoff, $SF_6$, $CF_4$ oder Edelgasen wie Helium, Neon, Argon, Krypton, insbesondere Stickstoff eingesetzt wird.

**11.** Verfahren nach mindestens einem der Ansprüche 7 bis 10, dadurch gekennzeichnet, daß die Fluorierung mit einem $N_2/F_2$-Gemisch durchgeführt wird, das zwischen 1 und 15 Vol.-%, insbesondere 2 und 10 Vol.-%, bevorzugt 3 bis 6 Vol.-% $F_2$ enthält.

**12.** Verfahren nach mindestens einem der Ansprüche 7 bis 11, dadurch gekennzeichnet, daß als Lösungsmittel Halogenkohlenwasserstoffe, insbesondere Dichlormethan, Chloroform, Tetrachlorkohlenstoff, Fluortrichlormethan, Trifluor-Trichloräthan, Tetrafluor-Dichloräthan oder Nitrile, insbesondere Acetonitril verwendet werden.

**13.** Verfahren nach mindestens einem der Ansprüche 7 bis 12, dadurch gekennzeichnet, daß die Fluorierung bei Temperaturen von -80 bis +10 °C, insbesondere -80 bis -20 °C, bevorzugt -60 bis -30 °C, besonders bevorzugt -50 bis -35 °C durchgeführt wird.

**14.** Verwendung von Verbindungen der Formel (I) zur Fluorierung von Verbindungen die offenen oder versteckten Carbanioncharakter besitzen.

**15.** Verwendung nach Anspruch 14, dadurch gekennzeichnet, daß als Verbindungen mit Carbanioncharakter 1,3-Dicarbonylverbindungen eingesetzt werden.

Europäisches Patentamt

# EUROPÄISCHER RECHERCHENBERICHT

Nummer der Anmeldung
EP 95 10 2962

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,Y | US-A-4 479 901 (BARNETTE W.E.) 30.Oktober 1984<br>* das ganze Dokument *<br>--- | 1,7,14 | C07D291/08<br>C07B39/00 |
| Y | EP-A-0 211 578 (RESEARCH CORPORATION) 25.Februar 1987<br>* das ganze Dokument * | 1,7,14 | |
| D | & US-A-4 828 764 (DESMARTEAU D.D.) 9.Mai 1989<br>--- | | |
| D,Y | TETRAHEDRON LETTERS,<br>Bd. 29, Nr. 47, 1988<br>Seiten 6087-6090,<br>DIFFERDING E. & LANG R.W. 'New fluorinating reagents - I- The first enantioselective fluorination reaction'<br>* das ganze Dokument *<br>--- | 1,7,14 | |
| Y | EP-A-0 311 086 (CIBA-GEIGY AG) 12.April 1989<br>* das ganze Dokument *<br>--- | 1,7,14 | **RECHERCHIERTE SACHGEBIETE (Int.Cl.6)** |
| Y | FURIN G.G. '2. Some "electrophilic" fluorination agents. In: New fluorinating agents in organic synthesis (German L. & Zemskov S., Eds.)'<br>1989 , SPRINGER-VERLAG , BERLIN ...<br>* Seite 35 - Seite 68 *<br>--- | 1,7,14 | C07D<br>C07B |
| Y | YUKI GOSEI KAGAKU KYOKAISHI (JOURNAL OF SYNTHETIC CHEMISTRY, JAPAN),<br>Bd. 50, Nr. 4, April 1992<br>Seiten 338-346,<br>UMEMOTO T. 'Recent development of fluorinating agents'<br>* Seite 342 - Seite 344 *<br>---<br>-/-- | 1,7,14 | |

Der vorliegende Recherchenbericht wurde für alle Patentansprüche erstellt

| Recherchenort | Abschlußdatum der Recherche | Prüfer |
|---|---|---|
| MÜNCHEN | 21.Juli 1995 | Hartrampf, G |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung
EP 95 10 2962

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | THE JOURNAL OF ORGANIC CHEMISTRY, Bd. 57, Nr. 15, 17.Juli 1992 Seiten 4281-4284, RESNATI G. & DESMARTEAU D.D. 'Electrophilic fluorination of pharmacologically active 1,3-dicarbonyl compounds' * das ganze Dokument * | 1,7,14 | |
| Y | US-A-5 254 732 (DIFFERDING E.) 19.Oktober 1993 * das ganze Dokument * | 1,7,14 | |
| A | DE-A-22 28 423 (FARBWERKE HOECHST AG, VORMALS MEISTER LUCIUS & BRÜNING) 20.Dezember 1973 * das ganze Dokument * | 7 | |
| D,A | ANGEWANDTE CHEMIE, Bd. 85, Nr. 22, November 1973 Seiten 965-973, CLAUSS K. & JENSEN H. 'Oxathiazinondioxide, eine neue Gruppe von Süsstoffen' * das ganze Dokument * | 7 | RECHERCHIERTE SACHGEBIETE (Int.Cl.6) |

Der vorliegende Recherchenbericht wurde für alle Patentansprüche erstellt

| Recherchenort | Abschlußdatum der Recherche | Prüfer |
|---|---|---|
| MÜNCHEN | 21.Juli 1995 | Hartrampf, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)